# EUROPÄISCHE PATENTSCHRIFT

(11) **EP 2 699 185 B1**
(45) Veröffentlichungstag und Bekanntmachung des Hinweises auf die Patenterteilung: **15.08.2018**
(21) Anmeldenummer: 12723098.5
(22) Anmeldetag: 21.04.2012
(51) Int. Cl.: A61B 90/14, A61B 6/04, A61B 90/00

(54) **PATIENTENFIXIERUNGSVORRICHTUNG MIT ADHÄSIVSCHICHT**
PATIENT-FIXING DEVICE WITH ADHESIVE LAYER
DISPOSITIF DE FIXATION POUR PATIENT COMPORTANT UNE COUCHE ADHÉSIVE

(30) Priorität: 21.04.2011 DE 202011005573 U
(43) Veröffentlichungstag der Anmeldung: 26.02.2014
(73) Patentinhaber: Isys Medizintechnik GmbH, 6370 Kitzbühel (AT)
(72) Erfinder: VOGELE, Michael, 86830 Schwabmünchen (DE)
(74) Vertreter: Fiener, Josef
(86) Internationale Anmeldenummer: PCT/EP2012/001730
(87) Internationale Veröffentlichungsnummer: WO 2012/143142

(56) Entgegenhaltungen:
- EP-A1- 2 272 433
- EP-A2- 1 166 740
- DE-A1-102005 006 775
- FR-A2- 2 631 547
- US-B1- 6 251 065
- US-B1- 6 533 793

## Beschreibung

Die Erfindung betrifft eine Vorrichtung zur Fixierung nach den oberbegrifflichen Merkmalen des Anspruches 1. Unter "Fixierung" sollen hierbei im medizinischen Sinne auch die Immobilisierung, Ruhigstellung, Schienung, Positionierung, Umlagerung, Komprimierung oder Abformung des menschlichen Körpers bzw. Teile des menschlichen Körpers verstanden werden, wobei insbesondere auch medizinische Zielvorrichtungen, Marker und/oder chirurgische Instrumenten für bildgestützte, minimalinvasive Operationsverfahren zuverlässig fixiert werden sollen.

In vielen Bereichen der Humanmedizin oder der medizinischen Forschung ist eine sichere Fixierung/Immobilisierung/Ruhigstellung/Schienung bzw. eine Komprimierung und oder Abformung des Patienten oder Teile des Patienten oder die (mechanische) Anbringung von Vorrichtungen oder Geräten erforderlich. Von größter Bedeutung ist dies insbesondere auf dem Gebiet der diagnostischen und therapeutischen Radiologie, der Strahlentherapie oder bei operativen/chirurgischen Eingriffen (Neurochirurgie, HNO, Orthopädie, usw.), aber auch bei der prä- oder postoperativen Versorgung von Wunden/Verletzungen.

Durch die Einbeziehung der Computertechnologie in Diagnose und Therapie sind die Anforderungen an Genauigkeit und Reproduzierbarkeit sowohl bei der Festlegung eines stereotaktischen Rahmensystems am Menschen als auch bei der Fixierung des Körpers selbst gestiegen. Komfort, Schnelligkeit in der Anwendung, Mobilität und Kosten spielen dabei eine erhebliche Rolle.

Als Stand der Technik sind folgende Fixationsarten bekannt:
a) Fixation des Körpers mit Bändern oder Manschetten:
   Der Körper des Patienten liegt hierbei auf einer Schaumstoffunterlage und quer über den Körper gespannte Bänder fixieren den Patienten auf diese Unterlage.
   Nachteilig sind dabei folgende Punkte:
      - Durch starken Zug der Bänder kann es zu Druckstellen, Verschiebungen und/oder Hautschwellungen kommen (keine homogene Druckverteilung);
      - nach Abnahme der Halteelemente (Bänder) ist eine erneute Repositionierung in genau gleicher Lage kaum mehr möglich, was besonders bei stereotaktischen Operationen und in der Strahlentherapie nachteilig ist;
      - der Körper ist nicht ausreichend fixierbar; besonders in seitlicher Richtung (nach lateral) ist die Beweglichkeit zu wenig einschränkbar oder definierbar.
b) Fixation des Körpers durch Verschraubung im Knochen:
   Der Körper des Patienten wird an mehreren Stellen über einen Metallrahmen verschraubt.
   Nachteilig ist dabei folgendes:
      - Die Verschraubung am Knochen stellt eine invasive Methode dar und ist somit nur bei bestimmten Indikationen möglich und gerechtfertigt;
      - die psychische Belastung des Patienten ist erheblich
      - die Methode ist nur auf bestimmte Lagerungen des Patienten anwendbar und behindert den Operateur;
      - eine Fixation der Weichteile (Muskel, Bänder, Bindegewebe) ist praktisch nicht möglich.
c) Fixation durch Schalungen :
   Hierbei wird der Patient auf eine Art "Luftmatratze" gelegt, welche mit Schaumstoffkügelchen gefüllt ist. Durch Absaugen der Luft in dieser Matratze verfestigt sich diese durch Aneinanderlegen der Schaumstoffkügelchen. Dabei wird die Vakuummatratze im ersten Schritt zunächst angepasst und im zweiten Schritt dann weiter abgesaugt. Durch diese Methode erhält man einen Abdruck des Körpers.
   Nachteilig ist hierbei:
      - die üblicherweise verwendeten "Matratzen" garantieren zwar eine Ruhigstellung, aber keine exakte Fixation;
      - der Körper wird allein durch die Schwerkraft oder durch Kompressionsbänder in Position gehalten, so dass keine fixe Verbindung zwischen den Oberflächen besteht;
      - bei unkooperativen Patienten ist eine ausreichende Ruhigstellung praktisch nicht möglich;
      - ebenso ist keine präzise Abformung möglich, da sich die Matratze in der Praxis nicht an allen Körperpartien exakt anlegen lässt;
      - durch Faltenbildung oder zu starkes Andrücken entstehen oftmals Druckstellen, welche vor allem bei narkotisierten Patienten zu Gewebeverletzungen führen können.
d) Vakuumfixationssystem:
   Bei Vakuumfixationssystemen wird das Abformelement über Vakuum an den Körper angelegt. Durch Absaugen der Luft zwischen Abformelement und Patientenoberfläche kann eine gute Verbindung für Abformung und Fixation geschaffen werden.
   Nachteilig hierbei ist:
      - Die Vakuumpumpe muss permanent laufen, damit die Verbindung zwischen Abformelement und Patient aufrecht erhalten wird;
      - Das System ist komplex, nicht leicht in der Handhabung und schlecht transportabel;
      - Bei Eingriffen, die hohe Keimfreiheit oder sogar Sterilität erfordern, stellt der Luftstrom der Vakuumpumpe eine Gefahr dar (Keimverschleppung);
      - Höhere Verbindungskräfte und damit bessere Abformung und Fixierung sind nicht möglich, weil durch zu hohe und lang anhaltende Drücke Verletzungen (z. B. Minderdurchblutung, Blutergüsse etc.) entstehen können;
      - Bei Ausfall des Vakuums geht die Verbindung verloren. Dies ist insbesondere dann kritisch, wenn z. B. bei Verlust der Verbindung eine Gefahr für Leib und Leben des Patienten entstehen kann oder der (chirurgische/radiologische) Eingriff abgebrochen bzw. wiederholt werden muss;
      - Das Verfahren ist insgesamt aufwendig und insbesondere für radiologische/chirurgische Eingriffe kaum praktikabel.

Andere Techniken wie Schienen, thermoplastisches Material, Kunststoffabdruck, Gipse, etc. weisen ähnliche Nachteile auf. Zusätzlich sind diese Methoden noch mit erheblichem finanziellen bzw. zeitlichen Aufwand verbunden und werden deshalb nur für Langzeitanwendungen verwendet.

Der Erfindung liegt deshalb die Aufgabe zugrunde, eine Vorrichtung zur Fixierung zu schaffen, welche die genannten Nachteile vermeidet, einfach in Aufbau und Anwendung und dabei in hohem Maße patientenschonend ist. Die Vorrichtung soll darüber hinaus die exakte Anbringung von Eichpunkten (sog. Markern) und eine optimale Zugänglichkeit zu Operationsgebieten ermöglichen.

Diese Aufgabe wird durch eine Vorrichtung mit den Merkmale des Anspruches 1 gelöst. Vorteilhafte Ausgestaltungen sind Gegenstand der Unteransprüche.

Ein wesentliches Merkmal ist hierbei eine Adhäsivschicht, mit der ein stabiles "Verkleben" des Abformelements am menschlichen Körper ermöglicht wird. Dies kann mit einem Sprühkleber oder einem Klebefilm erfolgen, so dass nach Abziehen eines Abdeckbandes ein Anpressen bzw. "Anmodellieren" der Adhäsivschicht möglich ist. Das Abformelement ist dabei noch weich, so dass eine exakte Anpassung erfolgen kann. Erst nach Anlegen von Vakuum am Abformelement wird dieses verfestigt und "erstarrt" zu einem formstabilen Abformelement mit stabiler Form.

Durch Arretierung eines Schwenkarms kann der fixierte Körper in Relation zu einer Grundplatte eines Eingriffs- oder Operationstisches zusätzlich fixiert werden, wobei - falls erwünscht - durch leichten Druck eine Kompression des Körpers erreicht wird. Die bevorzugt am Abformelement angebrachte Carbon-Adapterpiatte ist röntgentransparent und erhöht durch die hohe Steifigkeit die Stabilität der gesamten Vorrichtung.

Nach erfolgter Fixierung und steriler Abdeckung kann z. B. ein Eingriff (ggf. mit Roboterunterstützung) im OP-Fenster stattfinden. Der Roboter ist bevorzugt über die Grundplatte mechanisch an das Abformelement gekoppelt, also geometrisch festgelegt. Alternativ kann ein Mini-Roboter auch direkt am Abformelement z. B. über die Carbon-Adapterplatte angebracht sein. Ebenso können Marker für bildgebende Verfahren oder die Navigation reproduzierbar angebracht werden. Da es sich nicht um eine einfache, punktuelle Verklebung handelt, sondern um eine großflächige, ergonomische Anhaftung eines zunächst flexiblen, dann starren Abformelements, entsteht ein maximaler Oberflächenkontakt mit hohen Haltekräften bei maximalem Komfort.

Ausführungsbeispiele werden anhand der Zeichnungen nachfolgend beschrieben. Hierbei zeigen:
- Fig. 1 - 3: das Anbringen einer Vorrichtung zur Fixierung;
- Fig. 4: das Aufsetzen zusätzlicher Vorrichtungen;
- Fig. 5 und 6: das Abnehmen der Vorrichtung;
- Fig. 7 - 9: Ergänzungen der Vorrichtung zur Fixierung.

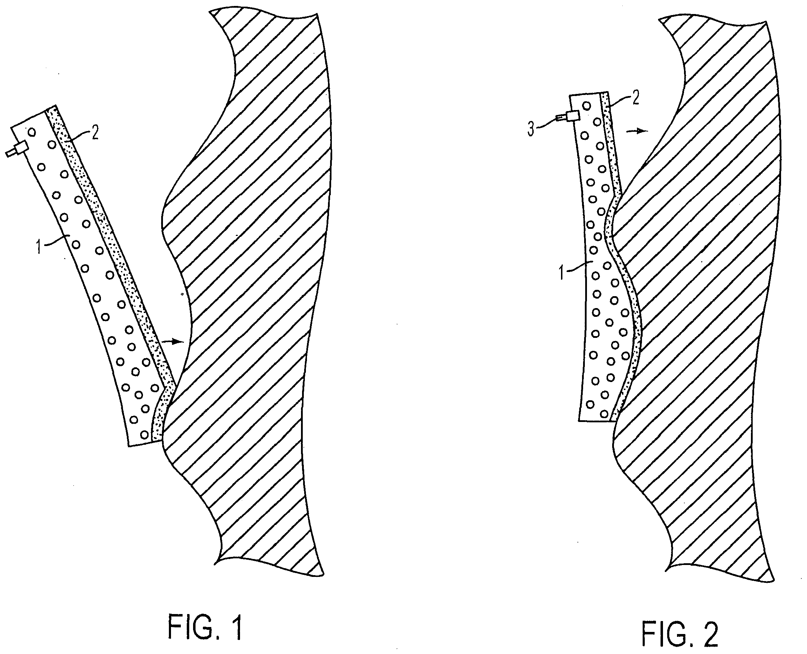

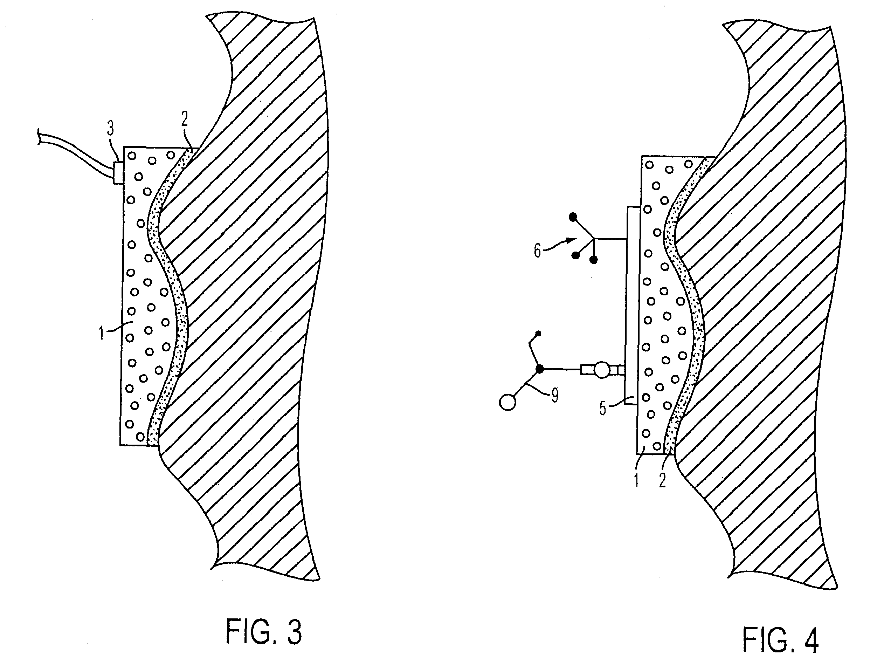

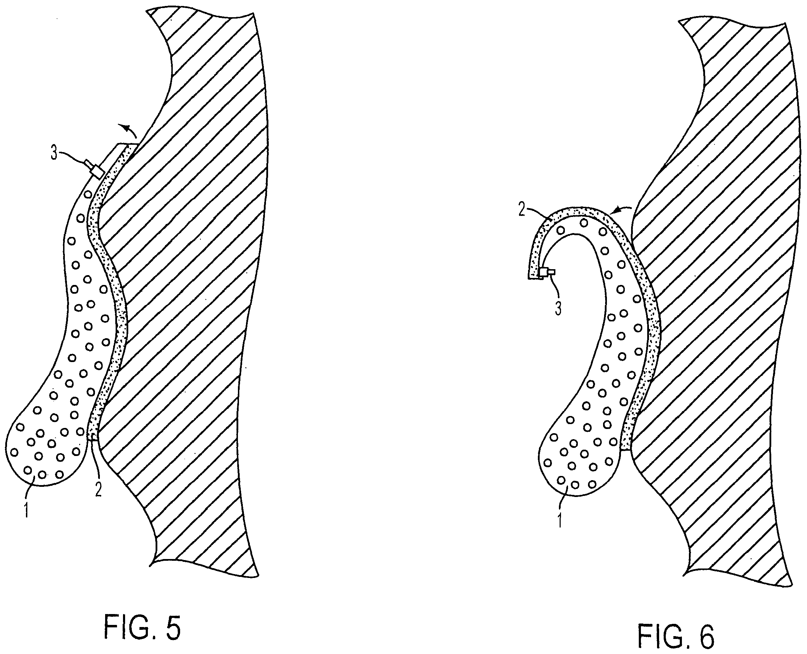

Die Vorrichtung zur Fixierung des menschlichen Körpers bzw. von Körperteilen besteht aus einem Abformelement 1, das auf der Körperoberfläche (schraffiert dargestellt; hier z. B. eine Rückenpartie) positionierbar ist (Fig. 1). Dies erfolgt mit einer Adhäsivschicht 2, die mit dem Abformelement 1 verbunden ist, z. B. einem inneren Klebefilm angeklebt ist und ggf. eine Trägerschicht zu einem äußeren Klebefilm aufweist, der an die Haut angedrückt wird. Die Adhäsivschicht 2 kann auch durch einen Sprühkleber gebildet sein, der auf die Haut und/oder die körperseitige Fläche des Abformelements 1 aufgesprüht wird. Wie aus Fig. 2 ersichtlich, wird das Abformelement 1 entlang der Wirbelsäule angedrückt und verklebt. Dann wird an einem Vakuumventil 3 eine Vakuumpumpe angeschlossen und das "Kissen" evakuiert. Hierdurch legen sich die Kügelchen im Abformelement eng aneinander und bilden so eine "harte", formstabile Schale. In dieser Position lässt sich das Abformelement 1 nicht abnehmen, sondern haftet mit hoher Haltekraft am Rücken oder im Schulterbereich. Erst nach Aufheben des Vakuums im Abformelement 1 lässt sich dieses wieder abziehen, wie in Fig. 4 und 5 schematisch dargestellt. Somit lässt sich das Abformelement 1 vom formstabilen Fixierungszustand wieder in einen weichen, formbaren Zustand zurückführen.

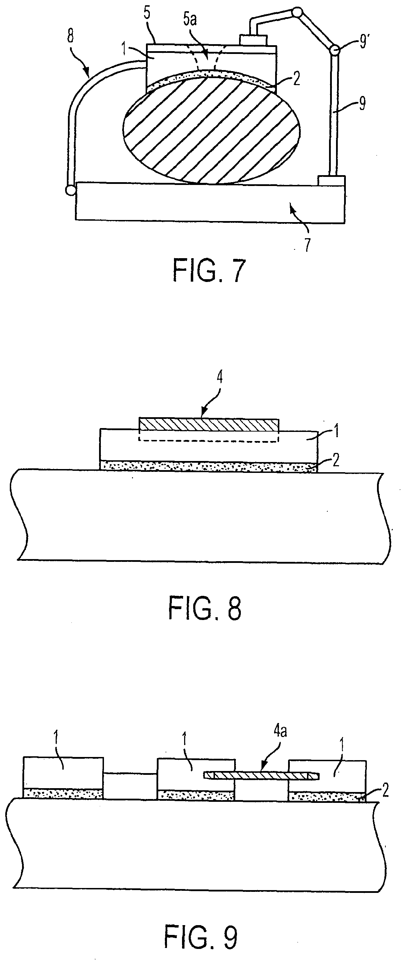

Die Hülle des Abformelements 1 ist gewebe- oder folienartig ausgebildet und mit Granulat, insbesondere Kunststoffkügelchen gefüllt ist. Das Abformelement 1 ist zum Andrücken an den Körper in Anbringrichtung elastisch und in Querrichtung zugfest ausgebildet ist. Wie in Fig, 4 dargestellt, ist an der Außenseite des verfestigten Abformelements 1 eine Adapterplatte 5, bevorzugt aus Carbon (CfK) angebracht, insbesondere verklebt oder mechanisch fixiert, um dort Marker 6 stabil und reproduzierbar zu verankern. Zur Fixierung an einer Grundplatte eines Eingriffs- oder Operationstisch 7 (vgl. Fig. 7) ist bevorzugt mindestens eine Schwenkarm-Aufhängung 9 vorgesehen.

Wie in Fig. 7 dargestellt, weist das Abformelement 1 zur zusätzlichen Fixierung am Randbereich Gurte bzw. Laschen 8 auf. Somit sind am Abformelement 1 diverse Zusatzeinrichtungen, insbesondere wenigstens eine Markervorrichtung 6 fixiert, vorzugsweise mittels wenigstens einer Adapterplatte 5 aus Carbon, in der ebenso wie im Abformelement 1 und der Adhäsivschicht 2 eine Eingriffsöffnung 5a (z. B, für eine Punktion im Lendenwirbelbereich) vorgesehen ist. Die Schwenkarm-Aufhängung 9 kann zudem wenigstens einen Kraft- oder Drehmomentsensor 9' aufweisen, um z. B. Bewegungen des Patienten zu erfassen und/oder zu begrenzen, und bei übermäßigen Bewegungen ein optisches Alarmsignal abzugeben. Damit lassen sich sogar Atembewegungen erfassen, so dass z. B. ein Mini-Roboter zum Führen einer Punktionsnadel entsprechend feinfühlig angesteuert werden kann.

Wie in Fig, 8 gezeigt, kann die als Vakuumkissen mit einem Vakuumventil 3 ausgebildete Vorrichtung außen am (oder im) Abformelement 1 mindestens eine Schiene 4, Insbesondere aus CfK zur Erhöhung der Steifigkeit aufweisen. Diese Gestaltung eignet sich insbesondere für die Anwendung an Armen oder Beinen. Mehrere Abformelemente 1 können z, B. am Rücken oder einem Bein auch mit Abstand zueinander angeordnet sein und bevorzugt über zugfeste Verbindungselemente 4a miteinander verbunden werden (vgl. Fig. 9). Hierdurch können entsprechende Operationsbereiche freigelassen werden. Bei großflächigen Vorrichtungen (vgl. Fig. 3 im gesamten Rückenbereich) weist das Abformelement 1) und die Adhäsivschicht 2 an wenigstens einer Stelle eine Operations- oder Eingriffsöffnung 5a auf. Die Abformelemente 1 können zum Beispiel L-förmig, C-förmig oder in Art eines Rechteckrahmens mit viereckigem oder bevorzugt kreisförmigem Durchbruch an der Operationsstelle gestaltet sein,

Somit kann der behandelnde Arzt oder sonstige Behandlungsperson praktisch jede Körperregion (ganzer Körper oder nur Körperteile) je nach Bedarf abformen und fixieren. Unterschiedliche Adapterplatten und OP-Fenstergrößen erhöhen die Flexibilität weiter. Somit ergibt sich eine beliebige Adaption an OP Tische und eine einfache Adaption von chirurgischen Geräten und Markern.

Zusätzlich denkbar ist:
- Unterpolsterung zwischen Adhäsivschicht und Abformelement (Erhöhung des Komforts)
- Poröse Adhäsivschicht und luftleitendes Zwischenelement ermöglicht Hautatmung (Schweißverdampfen und damit besserer Halt)
- Abkühlung/Erwärmung des Abformelements und damit möglicher therapeutischer Effekt bei z. B. Rücken-/Gelenksschmerzen

Insgesamt werden so neuartige Verfahren zur Fixierung/Ruhigstellung/Schienung von Körper- (teilen), insbesondere des menschlichen Körpers mit einem solchen Abformelement 1 mit wenigstens einer Adhäsivschicht 2 ermöglicht, welches durch Adhäsionskräfte an der Körperfläche angebracht wird. Da das Abformelement 1 mit Adhäsivschicht 2 durch Adhäsionskräfte am Körper angebracht wird, wird der umschlossene/eingeschlossene Körper mit definiertem Druck komprimiert und/oder exakt (re-) positioniert. Damit wird bei offenen Wunden oder Schnittverletzungen auch eine optimale Anlegung der Wundränder und Bildung eines "starren" Wundverbands (geringere Narbenbildung, bessere Heilung) erzielt.

## Patentansprüche

1. Vorrichtung zur Fixierung des menschlichen Körpers mit mindestens einem Abformelement (1), das auf der Körperoberfläche positionierbar ist und mindestens eine Adhäsivschicht (2) aufweist, wobei die Adhäsivschicht (2) des Abformelements (1) zur großflächigen, ergonomischen Anhaftung auf der Körperoberfläche positioniert ist und das Abformelement (1) an einem Eingriffs- oder Operationstisch (7) fixiert ist, insbesondere mittels einer Schwenkarm-Aufhängung (9), **dadurch gekennzeichnet, dass** das Abformelement (1) zur zusätzlichen Fixierung am Randbereich Gurte bzw. Laschen (8) aufweist.

2. Vorrichtung nach Anspruch 1, **dadurch gekennzeichnet, dass** das Abformelement (1) von einem formstabilen in einen formbaren Zustand überführbar ist, insbesondere durch Aufheben eines Vakuums im Abformelement (1),

3. Vorrichtung nach Anspruch 1 oder 2, **dadurch gekennzeichnet, dass** die Adhäsivschicht (2) aus einem inneren Klebefilm, einer Trägerschicht und einem äußeren Klebefilm besteht.

4. Vorrichtung nach einem der Ansprüche 1 bis 3, **dadurch gekennzeichnet, dass** die Hülle des Abformelements (1) gewebe- oder folienartig ausgebildet ist.

5. Vorrichtung nach einem der Ansprüche 1 bis 4, **dadurch gekennzeichnet, dass** die Hülle des Abformelements (1) mit Granulat, insbesondere Kügelchen gefüllt ist.

6. Vorrichtung nach einem der Ansprüche 1 bis 5, **dadurch gekennzeichnet, dass** das Abformelement (1) in Anbringrichtung elastisch und in Querrichtung zugfest ausgebildet ist.

7. Vorrichtung nach einem der Ansprüche 1 bis 6, **dadurch gekennzeichnet, dass** am Abformelement (1) wenigstens eine Markervorrichtung (6) oder medizinische Zielvorrichtung für bildgestützte Operationen angebracht ist und an einer Adapterplatte (5) auf der von der Körperoberfläche abgewandten Außenseite des Abformelements (1) fixiert ist.

8. Vorrichtung nach einem der Ansprüche 1 bis 7, **dadurch gekennzeichnet, dass** die Schwenkarm-Aufhängung (9) wenigstens einen Kraft- oder Drehmomentsensor (9') aufweist.

9. Vorrichtung nach einem der Ansprüche 1 bis 8, **dadurch gekennzeichnet, dass** das Abformelement (1) als Vakuumkissen mit einem Vakuumventil (3) ausgebildet ist.

10. Vorrichtung nach einem der Ansprüche 1 bis 9, **dadurch gekennzeichnet, dass** außen am oder im Abformelement (1) mindestens eine Schiene (4), insbesondere aus CfK zur Erhöhung der Steifigkeit vorgesehen ist.

11. Vorrichtung nach einem der Ansprüche 1 bis 10, **dadurch gekennzeichnet, dass** mehrere Abformelemente (1) mit Abstand zueinander angeordnet sind und bevorzugt über zugfeste Verbindungselemente (4a) miteinander verbunden sind.

12. Vorrichtung nach einem der Ansprüche 1 bis 11, **dadurch gekennzeichnet, dass** die Adhäsivschicht (2) durch einen hautverträglichen Sprühkleber gebildet ist.

13. Vorrichtung nach einem der Ansprüche 1 bis 12, **dadurch gekennzeichnet, dass** das Abformelement (1) und die Adhäsivschicht (2) sowie ggf. die Adapterplatte (5) an wenigstens einer Stelle eine Eingriffsöffnung (5a) aufweist.

## Claims

1. Device for fixation of the human body, having at least one molding element (1), which is adapted to be positioned on the body surface and has at least one adhesive layer (2), wherein the adhesive layer (2) of the molding element (1) is positioned for large-area, ergonomic adhesion on the body surface and wherein the molding element (1) is fixed to an intervention or operating table (7), in particular by a pivot arm suspension (9),
**characterized in that**
the molding element (1) has belts or tabs (8) at the edge area for additional fixation.

2. Device according to Claim 1, **characterized in that** the molding element (1) is transferable from a dimensionally-stable state into a moldable state, in particular by canceling a vacuum in the molding element (1).

3. Device according to Claim 1 or 2, **characterized in that** the adhesive layer (2) comprises an inner adhesive film, a carrier layer, and an outer adhesive film.

4. Device according to one of Claims 1 to 3, **characterized in that** the envelope of the molding element (1) is implemented like a fabric or film.

5. Device according to one of Claims 1 to 4, **characterized in that** the envelope of the molding element (1) is filled with granules, in particular beads.

6. Device according to one of Claims 1 to 5, **characterized in that** the molding element (1) is implemented as elastic in the attachment direction and as tension-resistant in the transverse direction.

7. Device according to one of Claims 1 to 6, **characterized in that** at least one marker device (6) or a medical targeting device for image-based operations is fixed to the molding element (1) and an adapter plate (5) at the outer, distal side of the molding element (1).

8. Device according to one of Claims 1 to 7, **characterized in that** the pivot arm suspension (9) has at least one force or torque sensor (9').

9. Device according to one of Claims 1 to 8, **characterized in that** the molding element (1) is implemented as a vacuum cushion having a vacuum valve (3).

10. Device according to one of Claims 1 to 9, **characterized in that** at least one splint (4), in particular made of CFRP to increase the stiffness, is provided externally on or in the molding element (1).

11. Device according to one of Claims 1 to 10, **characterized in that** multiple molding elements (1) are situated spaced apart from one another and are preferably connected to one another via tension-resistant connecting elements (4a).

12. Device according to one of Claims 1 to 11, **characterized in that** the adhesive layer (2) is formed by a skin-compatible spray adhesive.

13. Device according to one of Claims 1 to 12, **characterized in that** the molding element (1) and the adhesive layer (2) and optionally the adapter plate (5) have an engagement opening (5a) on at least one position.

## Revendications

1. Dispositif de fixation du corps humain avec au moins un élément de corsetage (1), qui peut être positionné sur la surface du corps et qui présente au moins une couche adhésive (2), dans lequel la couche adhésive (2) de l'élément de corsetage (1) est positionnée en vue de l'adhérence étendue ergonomique sur la surface du corps et l'élément de corsetage (1) est fixé à une table d'intervention ou d'opération (7), en particulier au moyen d'une suspension à bras pivotant (9), **caractérisé en ce que** l'élément de corsetage (1) présente dans la région du bord des ceintures ou des pattes (8) en vue d'une fixation supplémentaire.

2. Dispositif selon la revendication 1, **caractérisé en ce que** l'élément de corsetage (1) peut être amené d'un état de forme stable à un état déformable, notamment en supprimant un vide dans l'élément de corsetage (1).

3. Dispositif selon la revendication 1 ou 2, **caractérisé en ce que** la couche adhésive (2) se compose d'un film de colle intérieur, d'une couche de support et d'un film de colle extérieur.

4. Dispositif selon l'une quelconque des revendications 1 à 3, **caractérisé en ce que** la gaine de l'élément de corsetage (1) est réalisée sous forme de tissu ou de feuille.

5. Dispositif selon l'une quelconque des revendications 1 à 4, **caractérisé en ce que** la gaine de l'élément de corsetage (1) est remplie de granulat, en particulier de petites billes.

6. Dispositif selon l'une quelconque des revendications 1 à 5, **caractérisé en ce que** l'élément de corsetage (1) est élastique dans la direction d'application et résistant à la traction en direction transversale.

7. Dispositif selon l'une quelconque des revendications 1 à 6, **caractérisé en ce qu'**au moins un dispositif de marqueur (6) ou un dispositif de visée médical pour des opérations assistées par imagerie est installé sur l'élément de corsetage et est fixé à une plaque d'adaptateur (5) sur le côté extérieur de l'élément de corsetage (1) situé à l'opposé de la surface du corps.

8. Dispositif selon l'une quelconque des revendications 1 à 7, **caractérisé en ce que** la suspension à bras pivotant (9) présente au moins un détecteur de force ou de couple (9').

9. Dispositif selon l'une quelconque des revendications 1 à 8, **caractérisé en ce que** l'élément de corsetage (1) est formé par un coussin sous vide avec une soupape à vide (3).

10. Dispositif selon l'une quelconque des revendications 1 à 9, **caractérisé en ce qu'**il est prévu à l'extérieur ou dans l'élément de corsetage (1) au moins un rail (4), en particulier en CfK pour augmenter la rigidité.

11. Dispositif selon l'une quelconque des revendications 1 à 10, **caractérisé en ce que** plusieurs éléments de corsetage (1) sont disposés à distance l'un de l'autre et sont de préférence reliés l'un à l'autre par des éléments de liaison (4a) résistants à la traction.

12. Dispositif selon l'une quelconque des revendications 1 à 11, **caractérisé en ce que** la couche adhésive (2) est formée par une colle à pulvériser compatible avec la peau.

13. Dispositif selon l'une quelconque des revendications 1 à 12, **caractérisé en ce que** l'élément de corsetage (1) et la couche adhésive (2) ainsi qu'éventuellement la plaque d'adaptateur (5) présente une ouverture d'intervention (5a) en au moins un endroit.
